Europäisches Patentamt

European Patent Office

Office européen des brevets

(19)

(11) Publication number: **0 165 614**
A2

(12) **EUROPEAN PATENT APPLICATION**

(21) Application number: **85107679.4**

(22) Date of filing: **21.06.85**

(51) Int. Cl.⁴: **C 12 N 15/00**
C 12 N 1/20, C 12 N 9/38
//C12P21/02, (C12N1/20,
C12R1:19)

(30) Priority: **22.06.84 JP 127471/84**

(43) Date of publication of application:
**27.12.85 Bulletin 85/52**

(84) Designated Contracting States:
**DE FR GB**

(71) Applicant: **HITACHI, LTD.**
6, Kanda Surugadai 4-chome Chiyoda-ku
Tokyo 100(JP)

(72) Inventor: **Shimizu, Norio**
23-10, Hanayamacho-2-chome
Hitachi-shi(JP)

(72) Inventor: **Kurihara, Toshiharu**
Handai-Toneyamaryo C-204 1-11, Machikaneyamacho
Toyonaka-shi(JP)

(72) Inventor: **Masuda, Keiko**
Sun House Kaen 407 Higashiiru Sanjo Horikawa
Nakagyo-ku Kyoto(JP)

(72) Inventor: **Odawara, Yoji**
21-6, Nishinarusawacho-4-chome
Hitachi-shi(JP)

(72) Inventor: **Beppu, Teruhiko**
5-21, Horinouchi-1-chome Suginami-ku
Tokyo(JP)

(74) Representative: **von Füner, Alexander, Dr. et al,**
Patentanwälte v. Füner, Ebbinghaus, Finck Mariahilfplatz
2 & 3
D-8000 München 90(DE)

(54) **Hybrid plasmid and microorganism containing the same.**

(57) A hybrid plasmid comprising a tryptophan promoter and β-galactosidase gene, e.g., pTREZI, contained in a host microorganism such as *E. coli* can produce β-galactosidase in large amounts and can be used for searching new promoters or for detecting the expression activity of foreign genes.

FIG. 1

5'
CTCAAGGCGCACTCCCGTTCTGGATAATGTTTTTTGCGCCGACATCATAA

TaqI
CGGTTCTGGCAAATATTCTGAAATGAGCTGTTGACAATTAATCATCGAAC

Hpal  Rsal                          TaqI
  | PB |                        SD |    Leader
TAGTTAACTAGTACGCAAGTTCACGTAAAAAGGGTATCGACAATGAAAGC
                                        MetLysAl

peptide  Rsal
AATTTTCGTACTGAAAGGTTGGTGGCGCACTTCCTGAAACGGGCAGTGTA
aIlePheValLeuLysGlyTrpTrpArgThrSer

TTCACCATGCGTAAAGCAATCAGATACCCAGCCCGCCTAATGAGCGGGCT

          SD        trp E polypeptide
TTTTTTTGAACAAAATTAGAGAATAACAATGCAAACACAAAAACCGACTG
                        MetGlnThrGlnLysProThrG

EcoRI
  |                3'
GAATTCTC
LyIleLeu

Croydon Printing Company Ltd.

HYBRID PLASMID AND MICROORGANISM CONTAINING THE SAME

BACKGROUND OF THE INVENTION

This invention relates to a hybrid plasmid comprising a tryptophan promoter and β-galactosidase gene, and a host microorganism containing or harboring the hybrid plasmid and being capable of producing β-galactosidase.

Recently, there has been developed a recombinant DNA technique wherein a host microorganism such as Escherichia coli containing a hybrid plasmid is prepared by inserting a DNA having information for producing a useful substance such as human interferon, insulin, or the like into a vector plasmid of the host microorganism, and then subjected to the production of the useful substance in a large scale.

β-Galactosidase (β-gal) is an enzyme hydrolyzing lactose into glucose and galactose and is used for modifying milk for persons suffered from alactosia. β-Galactosidase is produced industrially from Escherichia coli (E. coli) Aspergillus niger and Aspergillus foetidus, but not satisfactory.

SUMMARY OF THE INVENTION

It is an object of this invention to provide a hybrid plasmide which can produce β-galactosidase in a large amount. It is another object of this invention to provide a hybrid plasmide which is useful for searching new promotors, or for detecting the production of foreign genes by means of expression of β-galactasidase. It is a further object of this invention to provide a process for preparing such a hybrid plasmid. It is a still further object of this invention to provide a host microorganism containing such a hybrid plasmid. It is also an object of this invention to provide a process for producing β-galactosidase.

This invention provides a hybrid plasmid comprising a tryptophan (trp) promoter and β-galactosidase (β-gal) gene connected to a DNA coding 8 amino acids at an N-terminal side of tryptophane E polypeptide on the downstream side of the trp promoter.

This invention also provides a process for producing hybrid plasmid pTREZl which comprises

(a) digesting plasmid pTREl with EcoRI and SalI to obtain a DNA fragment containing a tryptophan promoter (1),

(a') purifying the DNA fragment containing the tryptophan promoter (1) by an agarose gel electrophoresis,

(b) digesting plasmid pMC 1403 with EcoRI and SalI to obtain β-galactosidase gene,

(b') purifing the DNA fragment containing β-galactosidase gene by an agarose gel electrophoresis, and

(c) ligating the DNA fragment (1) and β-galactosidase gene with $T_4$ DNA ligase.

This invention further provides a microorganism containing a hybrid plasmid comprising a trp promoter and β-gal gene connected to a DNA coding 8 amino acids at an N-terminal side of trp E polypeptide on the downstream side of the trp promoter.

This invention also provide a process for producing β-galactosidase which comprises

cultivating E. coli containing hybrid plasmid pTREZ1 in a culture medium,

adding an inducer during the cultivation to induce the production of β-galactosidase,

recovering the E. coli,

lysing the recovered E. coli followed by crushing,

extracting β-galactosidase out of the cells of E. coli, and

purifying the extracted β-galactosidase.

BRIEF DESCRIPTION OF THE DRAWINGS

Fig. 1 shows a base sequence in a trp promoter.

Fig. 2 is a schematic diagram showing the construction of plasmid pTREZ1.

Fig. 3 shows a base sequence of a connecting portion of trp promoter and β-galactosidase gene.

DESCRIPTION OF THE PREFERRED EMBODIMENTS

The hybrid plasmid comprising a trp promoter and β-gal gene connected to a DNA coding 8 amino acids at an N-terminal side of trp E polypeptide on the downstream side of the trp promoter, more concretely pTREZ1, can be prepared from a plasmid containing a trp promoter and a plasmid containing β-gel gene therein.

The plasmid containing a trp promoter includes, for example, plasmid pTRE1, which is obtained by inserting a DNA fragment of about 500 bp (base pairs) comprising a promoter of trp operon of E. coli, trp L (leader peptide) and a part of terminal portion of trp E (an anthranilate synthase) into the EcoRI site of plasmid pBR322 [Gene, 29, 41-49 (1984)]. The direction of trp promoter is the direction of Tc$^r$ (a tetracycline resistance gene) of pBR322. Further, the EcoRI site on the upstream side of trp promoter is lacked by filling up with DNA polymerase and only the EcoRI site on the downstream side of trp promoter is improved.

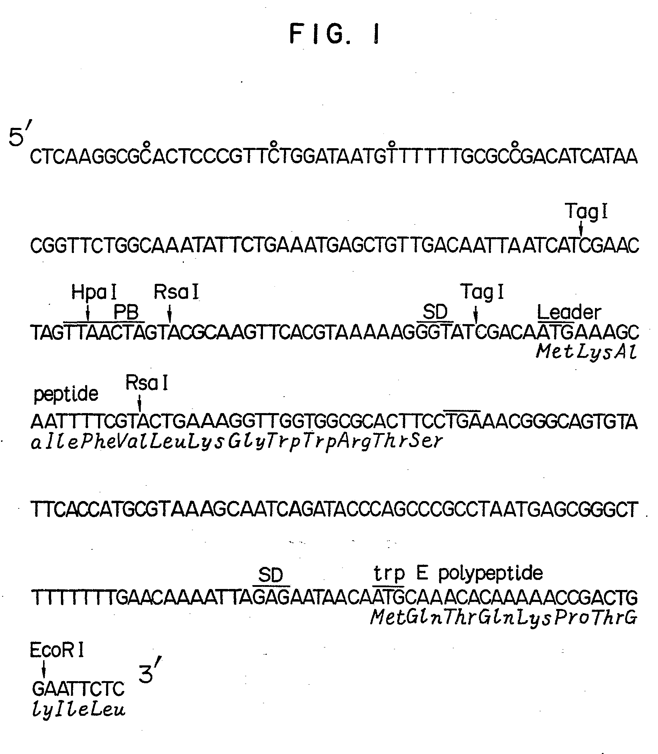

Fig. 1 shows a base sequence in the trp promoter. As is clear from Fig. 1, it is possible to express a foreign gene in the fused form with the 8 amino acids at the N-terminal side of trp E polypeptide by ligating the foreign gene at the EcoRI site in trp E polypeptide gene.

β-Galactosidase gene is available in the form of plasmid pMC1403 [J. Bacterol., 143, 971-980 (1980)]. Plasmid pMC1403 is obtained by inserting 6.2 kb (kiobase pairs) β-galactosidase gene (lac Z + lac Y) between the sites of EcoRI and SalI of pBR322.

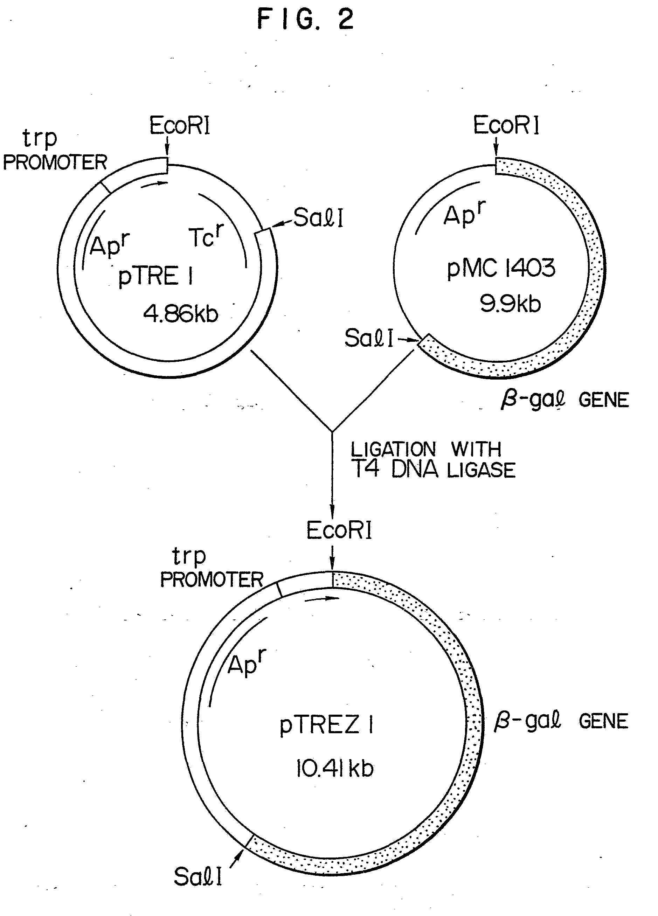

Fig. 2 is a schematic diagram showing the construction of plasmid pTREZ1 from plasmid pTRE1 and plasmid pMC1403. Plasmid pTRE1 is digested with restriction endonucleases EcoRI and SalI to give 4.21 kb DNA fragment (1) including trp promoter, followed by purification by a conventional method, e.g. agarose gel electrophoresis.  On the other hand, 6.2 kb of β-galactosidase gene is cleaved from pMC1403 by digestion with restriction endonucleases EcoRI and SalI, followed by purification by a conventional method, e.g. agarose gel electrophoresis.  The β-galactosidase gene and the DNA fragment (1) are ligated with $T_4$ DNA ligase to give 10.21 kb of hybrid plasmid pTREZl.

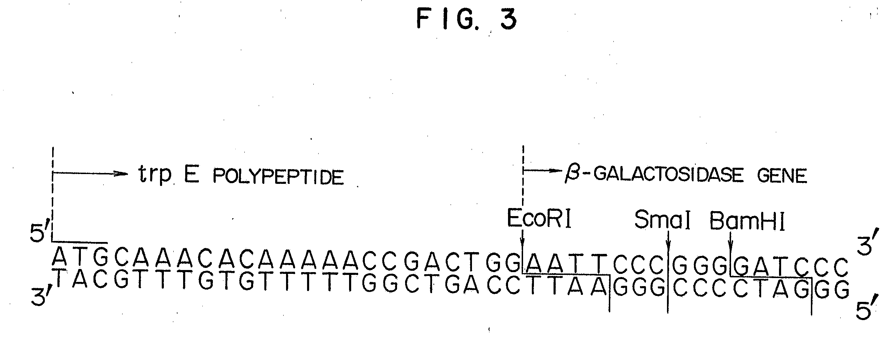

Fig. 3 shows a base sequence of a connecting portion of trp promoter and β-galactosidase gene on the hybrid plasmid.  As shown in Fig. 3, β-galactosidase gene is ligated to the DNA fragment coding 8 amino acids on the N-terminal side of trp E polypeptide.  Since there are restriction endonuclease sites of EcoRI, SmaI and BamHI near the connecting portion, it becomes possible to insert a foreign gene into these sites.  It is also possible to search new promotors other than the trp promoter and to detect the expression efficiency of foreign genes by measuring β-galactosidase activity, measurement of expression activity of the foreign genes having been difficult, by using the hybrid plasmid, e.g. pTREZl characterized by the restriction maps shown in Figs. 2 and 3.

More in detail, in the case of searching promoters other than the trp promoter, when one of the above-mentioned three restriction endonuclease sites is used, a DNA fragment cut from a chromosome in E. coli, etc. with the same restriction endonuclease is inserted thereinto, and an increase of the β-galactosidase activity is detected to attain the object.

0165614

In the case of detecting expression of foreign genes, there have been employed a method of directly detecting enzymatic or physiological activity per se, an antigen-antibody reaction using an antibody labelled with $^{125}I$, and the like, but complicated procedures were necessary for these methods. Contrary to the known complicated methods, when the hybrid plasmid of this invention is used and a foreign gene is inserted into either of the above-mentioned restriction endonuclease sites, the expression efficiency of a foreign gene by the trp promoter can easily be estimated by measuring the β- galactosidase activity.

As host microorganisms, there can be used E. coli such as E. coli M182, E. coli HB101, E. coli C600, E. coli X1776, E. coli DH1, etc. pTREZ1-containing E. coli M182 is deposited in the FRI as FERM BP-816, and pTREZ1-containing E. coli HB101 is deposited in the FRI as FERM BP-815.

By cultivating E. coli containing hybrid plasmid pTREZ1 of this invention, it is possible to produce β-galactosidase in larger amounts compared with conventional processes. Such a process comprises cultivating the E. coli(pTREZ1) in a culture medium containing a substrate such as glucose and casamino acid, adding an inducer such as IA (3-β-indolylacrylic acid) during the cultivation to induce the production of β-galactosidase, accumulating the produced β-galactosidase in a large amount in the cells, recovering the cultivated E. coli by centrifugation or membrane filtration, lysing cell walls of the recovered E. coli with lysozyme, etc., crushing the cell walls by a sonicator or a French press, extracting β-galactosidase out of the cells, removing the cells by centrifugation or membrane filtration to separate the supernatant, and separating and purifying β-galactosidase by gel filtration or membrane filtration to give the desired product.

This invention is illustrated by way of the following Examples.

Example 1

After digesting each about 3 μg of plasmids pTRE1 and pMC1403 with each 25 units of restriction endonucleases EcoRI and SalI, respectively, DNA fragments were recovered by agarose gel electrophoresis. Each recovered DNA fragment was dissolved in a TEN buffer (0.02 M Tris-HCl, 1 mM EDTA and 0.02 M NaCl; pH 8.0) and ligated with 2.5 units of $T_4$ DNA ligase (a total amount 50 μl) to give hybrid plasmid pTREZ1 characterized by the restriction maps shown in Figs. 2 and 3.

When the resulting hybrid plasmid was tried to be inserted into E. coli M182 lacking in β-galactosidase gene on chromosomes, no transformant was obtained. On the other hand, when E. coli C600 or E. coli M182 was transferred with plasmid pBR322, the transformation efficiency was $1 \times 10^6$ cells per μg DNA and $7 \times 10^3$ cells per μg DNA. It was found from these results that E. coli M182 was about 1/100 in the transformation efficiency compared with E. coli C600.

Then, E. coli C600 was transformed to give transformants. Among the transformants, a transformant containing plasmid pTREZ1 was selected and cultivated to obtained pTREZ1. Using the thus obtained pTREZ1, E. coli M182 was transformed.

One transformant was obtained from about 0.1 μg of plasmid pTREZ1 by the transformation of E. coli M182. The resulting E. coli M182 containing plasmid pTREZ1 is deposited in the FRI as FERM BP-816.

Example 2

E. coli M182 containing plasmid pTREZ1 was cultivated in 10 ml of L-broth culture medium [trypton 10 g, yeast extract 5 g, glucose 1 g, NaCl 5 g, water 1 liter, pH 7.2] added with 50 μg/ml of ampicillin at 37°C for 15 hours. One ml of the culture solution was inoculated in 10 ml of M9-casamino acid culture medium [$NH_4Cl$ 1 g, $Na_2HPO_4$ 6 g, $KH_2PO_4$ 3 g, NaCl 5 g, $MgSO_4 \cdot 7H_2O$ 0.1 g, $CaCl_2 \cdot 2H_2O$ 15 mg, glucose 2 g, casamino acid 20 g, distilled water 1 liter, pH 7.0] added with 50 μg/ml of ampicillin at 37°C for 4 hours. After cultivation for one hour, 15 μg/ml of 3-β-indolylacrylic acid (IA) was added to start the transcription of mRNA as an inducing substance.

After 4 hours, β-galactosidase activity was measured. One ml of the culture solution was sampled and 15 ml of toluene was added thereto with vigorous stirring, followed by shaking at 37°C for 30 minutes. To this, 2 ml of 0.35 M phosphate buffer (pH 7.25) and 2 ml of 0.25 M lactose solution were added, and the resulting solution was shaked at 30°C for 40 minutes, followed by immersion in boiling water for 15 minutes to stop the enzymatic reaction. The amount of glucose in the solution was measured by a glucose analyzer YSI 23A (manufactured by Yellow Springs Instrument Co., Inc.). The enzymatic activity of β-galactosidase was evaluated by defining the amount of hydrolyzing 1 μmole of lactose for 1 minute as 1 unit (U).

On the other hand, after cultivation for one hour, 200 μg/ml of tryptophan (Trp) as an repressing substance was added to the culture medium and β-galactosidase activity was measured in the same way as mentioned above.

The results are shown in Table 1.

Table 1

| Run No. | Microorganism | Addition of inducer or repressing substance (μg/ml) | | β-Gal activity in culture solution (U/ml) | Usable cell number (cell/ml) | β-Gal molecule per cell (U/cell) |
|---|---|---|---|---|---|---|
| 1 | E. coli M182(pTREZ1) | | 0 | 0.24 | $5.0 \times 10^8$ | 8,000 |
| 2 | E. coli M182(pTREZ1) | IA | 15 | 1.22 | $2.4 \times 10^8$ | 86,000 |
| 3 | E. coli M182 | | 15 | 0 | $5.0 \times 10^8$ | 0 |
| 4 | E. coli M182(pTREZ1) | Trp | 200 | 0.12 | $5.1 \times 10^8$ | 3,900 |

As is clear from Table 1, when the inducing substance IA is added to the culture solution, the β-galactosidase activity per ml of the culture solution becomes 1.22 U, which value is about 5 times as large as the case of adding no IA.  The activity of 1 mg of pure β-galactosidase when measured by an orthonitrophenyl-galactoside (ONPG) method is 340 U and the activity value of β-galactosidase measured by the ONPG method is about 5 times as high as the case measured by using the lactose substrate.  When the amount of β-galactosidase is calculated by using these values and converted to the number of β-galactosidase molecules per cell, it becomes 86,000 molecules.  This means that producing amount of β-galactosidase by E. coli M182(pTERZ1) is 20 to 30 times as large as the maximum production of ordinary E. coli.  That is, a large amount of enzyme β-galactosidase can be produced by the recombinant DNA technique.

On the other hand, tryptophan (Trp) is added as an repressing substance, the production of β-galactosidase is a half of the case adding no IA to E. coli M182(pTREZ1).  This means that when tryptophan is added, a repressser is activated and bonded to an operator portion of the tryptophan promoter, so that bonding of RNA polymerase to the trp promoter is prevented.

The data in Table 1 clearly show that the hybrid plasmid containing β-galactosidase gene connected in the downstream of trp promoter can produce 86,000 molecules of β-galactosidase per cell, and that the expression of β-galactosidase gene is controlled by the trp promoter.

As mentioned above, by using the hybrid plasmid comprising a trp promoter and β-galactosidase gene, for example, pTREZ1, β-galactosidase can be produced in large amounts, new promoters can be reached, and the production of foreign genes can be detected by the expression of β-galactosidase.

WHAT IS CLAIMED IS:

1.    A hybrid plasmid comprising a tryptophan promoter and β-galactosidase gene connected to a DNA coding 8 amino acids at an N-terminal side of tryptophane E polypeptide on the downstream side of the tryptophan promoter.

2.    Hybrid plasmid pTREZ1 characterized as shown by the restriction map in Fig. 2 and by the base sequence in Fig. 3 of the drawings.

3.    A process for producing hybrid plasmid pTREZ1 which comprises

(a) digesting plasmid pTRE1 with EcoRI and SalI to obtain a DNA fragment containing a tryptophan promoter (1),

(a') purifing the DNA fragment containing the tryptophan promoter (1) by an agarose gel electrophoresis,

(b) digesting plasmid pMC 1403 with EcoRI and SalI to obtain β-galactosidase gene,

(b') purifing the DNA fragment containing β-galactosidase gene by an agarose gel electrophoresis, and

(c) ligating the DNA fragment (1) and β-galactosidase gene with $T_4$ DNA ligase.

4.    A microorganism containing or hanboring a hybrid plasmid comprising a tryptophan promoter and β-galactosidase gene connected to a DNA coding 8 amino acids at an N-terminal side of tryptophan E polypeptide on the downstream side of the tryptophan promoter.

5.    A microorganism according to Claim 4, wherein E. coli is used as a host microorganism.

6.      E. coli M182 (pTREZ1) having the deposit accession number FERM BP-816.

7.      E. coli HB101 (pTREZ1) having the deposit accession number FERM BP-815.

8.      A process for producing β-galactosidase which comprises

cultivating E. coli containing a hybrid plasmid in a culture medium,

adding an inducer during the cultivation to induce the production of β-galactosidase,

recovering the E. coli,

lysing the recovered E. coli followed by crushing,

extracting β-galactosidase out of the cells of E. coli, and

purifying the extracted β-galactosidase

9.      A process according to Claim 8, wherein the hybrid plasmid is pTREZ1 characterized as shown by the restriction map in Fig. 2 and by the base sequence in Fig. 3 of the drawings.

10.      A process according to Claim 8, wherein the E. coli host organism is E. coli M182 (pTREZ1) having the deposit accession number FERM BP-816 or E. coli HB101 (pTREZ1) having the deposition accession number FERM BP-815.

EPAA-32952.O

# FIG. I

5′
CTCAAGGCGCACTCCCGTTCTGGATAATGTTTTTTTGCGCCGACATCATAA

TagI
↓
CGGTTCTGGCAAATATTCTGAAATGAGCTGTTGACAATTAATCATCGAAC

Hpa I        Rsa I                                    Tag I
↓      PB    ↓                              SD   ↓      Leader
TAGTTAACTAGTACGCAAGTTCACGTAAAAAGGGTATCGACAATGAAAGC
                                                    MetLysAl

peptide     Rsa I
            ↓
AATTTTCGTACTGAAAGGTTGGTGGCGCACTTCCTGAAACGGGCAGTGTA
alIePheValLeuLysGlyTrpTrpArgThrSer

TTCACCATGCGTAAAGCAATCAGATACCCAGCCCGCCTAATGAGCGGGCT

                        SD          trp E polypeptide
TTTTTTTGAACAAAATTAGAGAATAACAATGCAAACACAAAAACCGACTG
                                    MetGlnThrGlnLysProThrG

EcoR I
↓
GAATTCTC  3′
lyIleLeu

EPAA-32952.0

# F I G. 2

EPAA-32952.0

# FIG. 3

trp E POLYPEPTIDE →   → β-GALACTOSIDASE GENE

EcoRI     SmaI   BamHI

5'
ATGCAAACACAAAAACCGACTGG AATTCCC GGG GATCCC 3'
3' TACGTTTGTGTTTTTGGCTGACCTTAA GGG CCCCTAG GG 5'